## Europäisches Patentamt

## European Patent Office

## Office européen des brevets

(19)

(11) **EP 0 614 868 B1**

(12) **EUROPÄISCHE PATENTSCHRIFT**

(45) Veröffentlichungstag und Bekanntmachung des Hinweises auf die Patenterteilung:
**25.09.1996 Patentblatt 1996/39**

(21) Anmeldenummer: **94103357.3**

(22) Anmeldetag: **05.03.1994**

(51) Int. Cl.[6]: **C07C 45/35**, C07C 47/22, C07C 45/37, C07C 45/34, C07C 51/25, C07C 253/26, B01J 35/00, B01J 37/02

(54) **Verfahren zur Herstellung von 3 bis 5 C-Atome aufweisenden alpha,beta-monoethylenisch ungesättigten Aldehyden, Carbonsäuren oder Nitrilen**

Process for the preparation of 3 to 5 C-atoms containing alpha, beta-monoethylenic unsaturated aldehydes, carboxylic acids and nitriles

Procédé pour la préparation d'aldéhydes, d'acides carboxyliques et de nitriles alpha, béta-monoéthyléniques insaturés contenant 3 à 5 atomes de carbone

(84) Benannte Vertragsstaaten:
**BE DE ES FR GB IT NL**

(30) Priorität: **10.03.1993 DE 4307509**

(43) Veröffentlichungstag der Anmeldung:
**14.09.1994 Patentblatt 1994/37**

(73) Patentinhaber: **BASF Aktiengesellschaft**
**67063 Ludwigshafen (DE)**

(72) Erfinder:
- **Neumann, Hans-Peter, Dr.**
**D-68307 Mannheim (DE)**
- **Herzog, Klaus, Dr.**
**D-67071 Ludwigshafen (DE)**

(56) Entgegenhaltungen:
**EP-A- 0 450 596**     **EP-A- 0 468 290**

## Beschreibung

Die vorliegende Erfindung betrifft ein Verfahren zur Herstellung von 3 bis 5 C-Atome aufweisenden $\alpha,\beta$-monoethylenisch ungesättigten Aldehyden, Carbonsäuren oder Nitrilen durch katalytische Gasphasenoxidation oder -ammonoxidation einer reduzierten Form der gewünschten $\alpha,\beta$-monoethylenisch ungesättigten Verbindung in einem mit katalytisch aktiven Multimetalloxiden beschickten Rohrbündelreaktor bei erhöhter Temperatur und einem Umsatz der Ausgangsverbindung bei einfachem Durchgang von > 90 mol-%.

Die gasphasenkatalytisch oxidative oder ammonoxidative Herstellung von 3 bis 5 C-Atome aufweisenden $\alpha,\beta$-monoethylenisch ungesättigten Aldehyden, Carbonsäuren oder Nitrilen aus geeigneten reduzierten Formen dieser Verbindungen (insbesondere aus den entsprechenden Monoolefinen) in mit katalytisch aktiven Multimetalloxiden beschickten Rohrbündelreaktoren bei erhöhten Temperaturen ist allgemein bekannt (vgl. z.B. DE-A 2 004 251, EP-A 83 189, EP-A 835, EP-A 178 364, DE-A 2 203 709, DE-A 2 513 405, DE-A 2 635 031, EP-A 293 859, DE-A 2 251 364 und DE-A 2 626 887). Von besonderer Bedeutung sind die auf diese Art und Weise erfolgenden Herstellungen der entsprechenden C$_3$- bzw. C$_4$-Verbindungen, d.h. von Acrolein (z.B. oxidativ aus Propen), Methacrolein (z.B. oxidativ aus iso-Buten oder tert.-Butanol), Acrylsäure (z.B. oxidativ aus Acrolein), Methacrylsäure (z.B. oxidativ aus Methacrolein), Acrylnitril (z.B. ammonoxidativ aus Propen oder Acrolein) und Methacrylnitril (z.B. ammonoxidativ aus iso-Buten oder tert.-Butanol).

Eine Vielzahl von für diese Umsetzungen geeigneten Multimetalloxidkatalysatoren läßt sich unter der allgemeinen Formel I

$$Mo_{12} Bi_a Fe_b X^1_c X^2_d X^3_e X^4_f O_n \qquad (I),$$

in der die Variablen nachfolgende Bedeutung aufweisen:

| | |
|---|---|
| $X^1$ | Nickel und/oder Kobalt, |
| $X^2$ | Thallium, ein Alkalimetall und/oder ein Erdalkalimetall, |
| $X^3$ | Phosphor, Arsen, Bor, Antimon, Zinn, Cer, Blei und/oder Wolfram, |
| $X^4$ | Silicium, Aluminium, Titan und/oder Zirkonium, |
| a | 0,5 bis 5, |
| b | 0,01 bis 3, |
| c | 3 bis 10, |
| d | 0,02 bis 2, |
| e | 0 bis 5, |
| f | 0 bis 10 und |
| n | eine Zahl, die durch die Wertigkeit und Häufigkeit der von Sauerstoff verschiedenen Element bestimmt wird, |

subsumieren.

Sie sind in an sich bekannter Weise erhältlich (siehe z.B. die ältere Anmeldung DE-A 40 23 239 und werden üblicherweise in Substanz zu Kugeln, Ringen oder Zylindern geformt oder auch in Gestalt von Schalenkatalysatoren, d.h. mit der Aktivmasse beschichteten vorgeformten inerten Trägerkörpern, eingesetzt. Selbstverständlich können sie aber auch in Pulverform als Katalysatoren angewendet werden.

Als Oxidationsmittel wird Sauerstoff verwendet (bei der Ammonoxidation ist zusätzlich die Anwesenheit von Ammoniak erforderlich), wobei die Reaktionspartner zweckmäßigerweise mit Inertgas wie N$_2$, rückgeführten Reaktionsabgasen und/oder Wasserdampf verdünnt werden. Unter diesem Aspekt erweist sich die Verwendung von Luft als Sauerstoffquelle besonders vorteilhaft. Üblicherweise werden die Reaktionen als Festbettoxidationen bzw. -ammonoxidationen geführt. Die diesbezüglichen Einzelheiten sind allgemeiner Stand der Technik.

Charakteristisch für diese gasphasenkatalytisch oxidativen oder ammonoxidativen Herstellungen ist, daß sie einerseits einer erhöhten Reaktionstemperatur bedürfen (diese liegt in der Regel im Bereich von 200 bis 600°C) und andererseits stark exotherm verlaufen. Normalerweise sind die die aktiven Multimetalloxide enthaltenden Kontaktrohre daher von einer zirkulierenden Flüssigkeit (z.B. eine Salzschmelze) als Temperiermedium umgeben.

Ferner ist es für diese gasphasenkatalytisch oxidativen oder ammonoxidativen Herstellungen charakteristisch, daß mit der eigentlichen Zielumsetzung eine Vielfalt von möglichen Konkurrenzreaktionen (z.B. Parallel- und Folgereaktionen) einhergeht, die die Zielproduktbildung mindern. Um eine möglichst selektive Zielproduktbildung zu erreichen, ist es daher erforderlich, die Reaktion zu steuern. Eine in der Fachwelt anerkanntermaßen besonders vorteilhafte Maßnahme zur selektiveren Zielproduktbildung bei vorgegebenem, möglichst hohem, Eduktumsatz besteht darin, die volumenspezifische Aktivität der katalytischen Beschickung (= Aktivität pro Volumen des Reaktionsraums) längs der Kontaktrohre in Strömungsrichtung zu erhöhen, so daß die volumenspezifische Aktivität von der Eintrittsseite des Reaktionsgases zur Austrittsseite hin zunimmt (vgl. z.B. DE-A 3 006 894, EP-A 441 312 oder DE-AS 2 056 614).

Als Gegenstand der vorliegenden Erfindung wurde nun in überraschender Weise festgestellt, daß, im Gegensatz zur gängigen Fachmeinung, eine verbesserte Zielproduktbildung bei den Verfahren zur Herstellung von 3 bis 5 C-Atome aufweisenden $\alpha,\beta$-monoethylenisch ungesättigten Aldehyden, Carbonsäuren oder Nitrilen durch katalytische Gasphasenoxidation oder -ammonoxidation einer reduzierten Form der gewünschten $\alpha,\beta$-monoethylenisch ungesättigten Verbindung (insbesondere aus den entsprechenden Monoolefinen) in einem mit katalytisch aktiven Multimetalloxiden beschickten Rohrbündelreaktor bei erhöhter Temperatur und einem Umsatz der Ausgangsverbindungen bei einfachem Durchgang von > 90 mol-% dann erhalten wird, wenn die volumenspezifische Aktivität der katalytischen Beschickung längs der Kontaktrohre in Strömungsrichtung abrupt, sukzessiv stufenweise und/oder kontinuierlich abnimmt. Dies gilt insbesondere für die gasphasenkatalytisch oxidative Herstellung der entsprechenden $C_3$- bzw. $C_4$-Verbindungen, d.h. von Acrolein (aus Propen), von Acrylsäure (aus Acrolein), Methacrolein (aus iso-Buten oder tert.-Butanol), Methacrylsäure (aus Methacrolein) sowie die von den entsprechenden Ausgangsverbindungen ausgehende ammonoxidative Erzeugung von (Meth) acrylnitril, insbesondere dann, wenn als Multimetalloxidmasse eine solche der allgemeinen Formel I eingesetzt wird und die Temperatur der zirkulierenden Temperierflüssigkeit 200 bis 600°C beträgt. Eine Abnahme der volumenspezifischen Aktivität ist beispielsweise dadurch realisierbar, daß man die Aktivmassenkonzentration reduziert oder eine höhere volumenspezifische Aktivität aufweisende Katalysatoren durch solche einer geringeren volumenspezifischen Aktivität ablöst (z.B. Änderung der Zusammensetzung der Multimetalloxide).

Beispiele

a) Herstellung eines Multimetalloxidkatalysators A
Eine feinteilige Multimetalloxidmasse der Zusammensetzung $Mo_{12}Bi_1Co_5Fe_{2,5}W_2Si_{1,6}K_{0,1}O_x$ wurde zu Hohlzylindern von 3 mm Länge, 5 mm Außendurchmesser und 1,5 mm Wanddicke gepreßt.

b) Herstellung eines Multimetalloxidkatalysators B
Die feinteilige Multimetalloxidmasse aus a) wurde in einer Schichtdicke von 400 μm auf inerte Trägerkugeln (aus Steatit) von 5 mm Durchmesser aufgetragen.

c) Katalytische Gasphasenoxidation von Propen zu Acrolein

1. In einem salzbadbeheizten Rohrreaktor (V2A, 2 mm Wandstärke, 25 mm Innendurchmesser) wurde ein Volumen von 1200 ml mit dem Multimetalloxidkatalysator A gefüllt und mit 2400 Nl/h einer Gasmischung der Zusammensetzung 5 Vol.-% Propen, 45 Vol.-% Luft und 50 Vol.-% Stickstoff beschickt. Bei einer konstanten Salzbadtemperatur von 330°C stellte sich ein Propenumsatz von 96 mol-% bei einer Selektivität von, bezogen auf die Gesamtmenge aus hauptsächlich gebildetem Acrolein und geringfügig gebildeter Acrylsäure als weiterem Wertprodukt (die Herstellung von Acrolein ist insbesondere als erste Oxidationsstufe zur nachfolgenden Oxidation des Acroleins zu Acrylsäure von Bedeutung) von 92 mol-% ein.

2. In einem identischen Rohrreaktor wie in c) 1. wurde ein Volumen von 1200 ml mit dem Multimetalloxidkatalysator B gefüllt und in gleicher Weise wie in c) 1. beschickt. Bei einer konstanten Salzbadtemperatur von 340°C stellte sich bei einem identischen Propenumsatz von 96 mol-% eine in gleicher Weise bezogene Selektivität von 91,5 mol-% ein.

Die für denselben Propenumsatz erforderliche höhere Salzbadtemperatur weist aus, daß die volumenspezifische Aktivität einer Beschickung mit dem Multimetalloxidkatalysator B geringer ist, als diejenige einer Beschickung mit dem Multimetalloxidkatalysator A.

3. In einem identischen Rohrreaktor wie in c) 1. wurde in Strömungsrichtung zunächst ein Volumen von 800 ml mit dem Multimetalloxidkatalysator B und sich daran unmittelbar anschließend ein Volumen von 400 ml mit dem Multimetalloxidkatalysator A gefüllt und in gleicher Weise wie in c) 1. beschickt. Bei einer konstanten Salzbadtemperatur von 329°C stellte sich bei einem identischen Propenumsatz von 96 mol-% eine in gleicher Weise bezogene Selektivität von 91,8 mol-% ein.

4. In einem identischen Rohrreaktor wie in c) 1. wurde erfindungsgemäß in Strömungsrichtung zunächst ein Volumen von 800 ml mit dem Multimetalloxidkatalysator A und sich daran anschließend ein Volumen von 400 ml mit dem Multimetalloxidkatalysator B gefüllt und in gleicher Weise wie in c) 1. beschickt. Bei einer konstanten Salzbadtemperatur von 330°C stellte sich bei einem identischen Propenumsatz von 96 mol-% eine in gleicher Weise bezogene Selektivität von 93 mol-% ein.

**Patentansprüche**

1. Verfahren zur Herstellung von 3 bis 5 C-Atome aufweisenden $\alpha,\beta$-monoethylenisch ungesättigten Aldehyden, Carbonsäuren oder Nitrilen durch katalytische Gasphasenoxidation oder -ammonoxidation einer reduzierten Form der gewünschten $\alpha,\beta$-monoethylenisch ungesättigten Verbindung in einem mit katalytisch aktiven Multimetalloxiden beschickten Rohrbündelreaktor bei erhöhter Temperatur und einem Umsatz der Ausgangsverbindungen bei einfachem Durchgang von > 90 mol-%, dadurch gekennzeichnet, daß die volumenspezifische Aktivität der katalytischen Beschickung längs der Kontaktrohre in Strömungsrichtung abrupt, sukzessive und/oder kontinuierlich abnimmt.

## Claims

1. A process for the preparation of $\alpha,\beta$-monoethylenically unsaturated aldehydes, carboxylic acids or nitriles having 3 to 5 carbon atoms by catalytic gas-phase oxidation or ammonoxidation of a reduced form of the desired $\alpha,\beta$-monoethylenically unsaturated compound in a tube-bundle reactor charged with catalytically active multimetal oxides at elevated temperature and at a conversion of starting compounds for a single pass of > 90 mol%, wherein the volume-specific activity of the catalytic charge drops abruptly, successively and/or continuously along the contact tubes in the flow direction.

## Revendications

1. Procédé de préparation de nitriles, d'acides carboxyliques, ou d'aldéhydes, $\alpha,\beta$-monoéthyléniquement insaturés et présentant de 3 à 5 atomes de carbone, par l'ammonoxydation ou l'oxydation catalytique en phase gazeuse d'une forme réduite du composé $\alpha,\beta$-monoéthyléniquement insaturé dans un réacteur à faisceau de tubes garni d'oxydes de métaux multiples catalytiquement actifs, à température élevée et avec une conversion des composés de départ pour un seul passage supérieur à 90% molaires, caractérisé en ce que l'activité volumique spécifique de la garniture catalytique diminue continuellement, successivement et/ou de manière abrupte le long des tubes de contact dans la direction d'écoulement.